(19) Europäisches Patentamt / European Patent Office / Office européen des brevets

(11) EP 1 591 123 B1

(12) EUROPEAN PATENT SPECIFICATION

(45) Date of publication and mention of the grant of the patent:
**19.08.2009 Bulletin 2009/34**

(21) Application number: **04701576.3**

(22) Date of filing: **13.01.2004**

(51) Int Cl.:
*A61K 31/70* (2006.01) *A61K 31/35* (2006.01)
*A61P 11/10* (2006.01) *A61P 11/14* (2006.01)

(86) International application number:
**PCT/CN2004/000041**

(87) International publication number:
**WO 2004/064848 (05.08.2004 Gazette 2004/32)**

# (54) USES OF NARINGENIN, NARINGIN AND SALTS THEREOF AS EXPECTORANTS IN THE TREATMENT OF COUGH, AND COMPOSITIONS THEREOF

VERWENDUNG VON NARINGENIN, NARINGIN UND SALZEN DARAUS ALS SCHLEIMLÖSER BEI DER BEHANDLUNG VON HUSTEN SOWIE ZUSAMMENSETZUNGEN DARAUS

UTILISATION DE NARINGENINE, DE NARINGINE ET DE LEURS SELS EN TANT QU'EXPECTORANTS DANS LE TRAITEMENT DE LA TOUX ET COMPOSITIONS CORRESPONDANTES

(84) Designated Contracting States:
**AT BE BG CH CY CZ DE DK EE ES FI FR GB GR HU IE IT LI LU MC NL PT RO SE SI SK TR**

(30) Priority: **21.01.2003 CN 03113605**
**08.01.2004 CN 200410015024**

(43) Date of publication of application:
**02.11.2005 Bulletin 2005/44**

(73) Proprietors:
- **Su, Wei-wei**
  **Guangzhou, Guangdong 510275 (CN)**
- **Wang, Yong-gang**
  **Guangzhou, Guangdong 510275 (CN)**
- **Fang, Tie-zheng**
  **Guangzhou, Guangdong 510275 (CN)**
- **Peng, Wei**
  **Guangzhou, Guangdong 510275 (CN)**
- **Wu, Zhong**
  **Guangzhou, Guangdong 510275 (CN)**

(72) Inventors:
- **Su, Wei-wei**
  **Guangzhou, Guangdong 510275 (CN)**
- **Wang, Yong-gang**
  **Guangzhou, Guangdong 510275 (CN)**
- **Fang, Tie-zheng**
  **Guangzhou, Guangdong 510275 (CN)**
- **Peng, Wei**
  **Guangzhou, Guangdong 510275 (CN)**
- **Wu, Zhong**
  **Guangzhou, Guangdong 510275 (CN)**

(74) Representative: **Beier, Ralph**
**v. Bezold & Partner**
**Patentanwälte**
**Akademiestrasse 7**
**80799 München (DE)**

(56) References cited:
**IL-A- 91 425** **US-A- 6 165 984**

- **TABAK C ET AL: "Chronic obstructive pulmonary disease and intake of catechins, flavonols, and flavones: the MORGEN Study." AMERICAN JOURNAL OF RESPIRATORY AND CRITICAL CARE MEDICINE. 1 JUL 2001, vol. 164, no. 1, 1 July 2001 (2001-07-01), pages 61-64, XP002364706 ISSN: 1073-449X**
- **DATABASE BIOSIS [Online] BIOSCIENCES INFORMATION SERVICE, PHILADELPHIA, PA, US; 1980, LAMBEV I ET AL: "ANTI EXUDATIVE EFFECT OF NARINGIN ON THE EXPERIMENTAL LUNG EDEMA AND PERITONITIS" XP002364708 Database accession no. PREV198273012319**
- **KNEKT PAUL ET AL: "Flavonoid intake and risk of chronic diseases" AMERICAN JOURNAL OF CLINICAL NUTRITION, vol. 76, no. 3, September 2002 (2002-09), pages 560-568, XP002364727 ISSN: 0002-9165**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 591 123 B1

- JINGSU NEW MEDICAL COLLEGE ED.: 'Cyclopedia of chinese herbs', vol. 1, 1986, SHANGHAI SCIENTIFIC AND TECHNIC PRESS pages 1505 - 1506

## Description

### FIELD OF THE INVENTION

This invention relates to the use of Naringenin, Naringin, and their salts for the preparation of a medicament for the treatment of cough and dispelling phlegun (mucus)

### BACKGROUND OF THE INVENTION

Cough, sputum and asthma are common indications of respiratory diseases clinically with cough and expectoration, which are usually caused by acute or chronic bronchitis. Infants and middle aged and elderly people easily fall cough and expectoration caused by acute or chronic bronchitis because of their constitution. As a common encountered disease, it may get worse and have a severe impact on human health and life quality if it is not correctly treated and controlled early.

As for chemical medicines used in relieving cough and dispelling phlegm, medicines for relieving cough include codeine, dextromethorphan, pentoxyverine, benproperine etc; medicines for dispelling phlegm include ammonium chloride, acetylcysteine, bromhexine etc. A shortage in common of both medicines is: the former, though it can cure cough without sputum, has no effect on dispelling phlegm, so it is not suitable for cough with sputum; while the latter can hardly cure cough. In addition, side-effects of chemical medicines are serious, for example, codeine may cause addiction with long-term usage; dextromethorphan easily arouse dizziness, belching; pentoxyverine can cause mild headache, dizziness, thirsty, astriction, atropine-liked effects and is forbidden for glaucoma patients; benproperine may cause untoward reaction such as dizziness, stomach burning and tetter; ammonium chloride can locally excite stomach and must be used in canker and hepatic and renal incapacity patients with extra caution; acetylcysteine with special fetor may cause nausea and is unsuitable for bronchitis patients because of its excitation to respiratory system; bromhexine is unsuitable for peptic ulcer patients with a probability of stomach unease.

People have paid much effort to Traditional Chinese Medicine (TCM) for relieving cough and dispelling phlegm. Some of these complex prescription made from TCM, such as Juhong Pills, Chuanbei-Pipa Syrup and Xiaokechuan Syrup have been embodied in China Pharmacopoeia I (2000). But in terms of their characteristic, these medicines have the shortage of discommodiousness with highdose. The Juhong Pill, composed of 15 kinds of botanic materials such as Huajuhong, Chenpi, is taken over 10g per dose; the Chuanbei-Pipa Syrup is made from Chuanbeimu, Jiegeng, Pipa leaves, Bohenao and the Xiaokechuan Syrup is made from Manshanhong. Both need 10ml per dose. All of the three preparations are inconvenient when schlepping and taking.

As a result, it’s necessary to keep on researching and developing a new kind of medicine for both cough with or without sputum, which has no excitation to stomach and respiratory system, tiny side-effects and is convenient to schlep and take, safe, and effective.

Naringenin, 4’, 5, 7 -trihydroxyflavanone, is the aglycon of the Naringin, a kind of flavonoids. Naringenin, also a kind of flavonoids, widely exists in the material tissues such as buds of cherry and plum. It can inhibit oxidation, resist ulcer, inhibit breast cancer hyperplasia, delay the formation of breast tumor, and treat cardiovascular diseases. Naringin, the ramification of Naringenin, 4’, 5’, 7-trihydroxyflavanone-7-rhamnoneglucoside, has been reported to inhibit oxidation, resist inflammation and ulcer. It hasn’t been reported that the Naringenin, Naringin and salts thereof have the effects of relieving cough and dispelling phlegm.

Knekt et al. (American Journal of Clinical Nutrition, vol. 76, no. 3. (2002), 560-568) disclose that flavonoids and, i.a., Naringenin, are effective antioxidants which have a preventive action and are able to reduce the incidence of asthma but do neither disclose nor suggest that Naringin/Naringenin has the specific property of dispelling phlegm (mucus) and may be used for preparing a medicament for the treatment of an acute disorder characterized by cough and excess mucus.

Similarly, Tabac et al. (American Journal of Respiratory and Critical Care Medicine, vol 164, no. 1 (2001), 61-64) investigate the effects of the intake of catechines, flavonols and flavones in order to protect from a chronic obstructive pulmonary disease, but do not mention the use of Naringenin or Naringin for this purpose or for the treatment of an acute disorder.

US-A-6 165 984 discloses pharmaceutical compositions comprising Naringenin or Naringin for treating hepatic diseases but not for the treatment of cough.

Molecular formula of Naringenin: $C_{15}H_{12}O_5$

Structural formula of Naringenin:

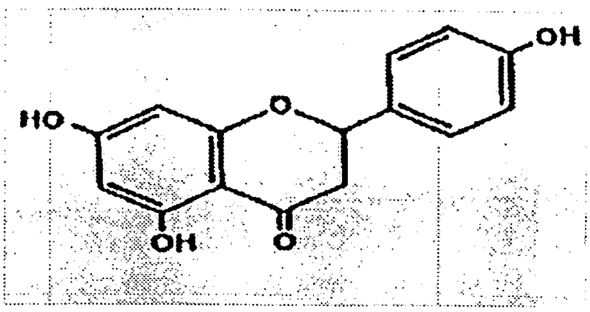

Naringenin

Molecular formula of Naringin: $C_{27}H_{32}O_{14}$

Structural formula ofNaringin:

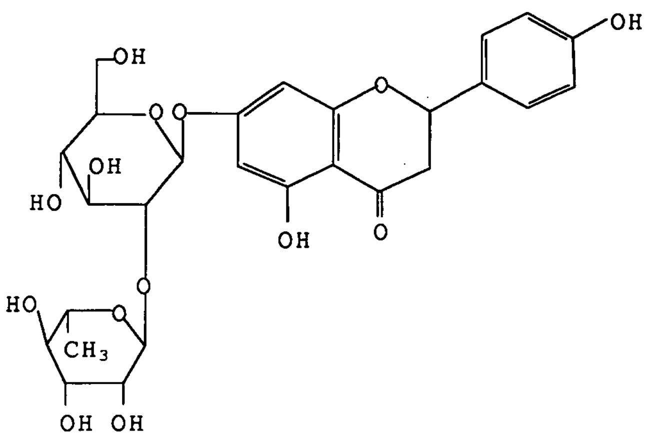

Naringin

Because both Naringenin and Naringin are flavonoids with free penol carbonyl, they can form salts with alkalescent substrates, such as NaOH, KOH, $NaHCO_3$, $KHCO_3$, $Na_2CO_3$ and $K_2CO_3$.

The salts of Naringenin mean that Naringenin reacts with the alkalescent bubstrantes mentioned above to form sodium or potassium salts. The salts of Naringin mean that Naringin reacts with the alkalescent bubstances mentioned above to form sodium or potassium salts.

SUMMARY OF THE INVENTION

The object of this invention is to provide a new therapeutic use of Naringenin, Naringin and salts thereof. The use mentioned in the invention is the use of Naringenin, Naringin and salts thereof in preparation of pharmaceutical compositions for the treatment of cough and dispelling phlegm (mucus).

A related object of this invention is to provide pharmaceutical compositions for the treatment of cough and dispelling phlegm mucus.

The pharmaceutical compositions contain effective amount of Naringenin, Naringin and salts thereof as active ingredients, and pharmaceutically acceptable carriers.

The effective amount of Naringenin, Naringin and salts thereof is 0.1~500 mg/kg(avoirdupois)/day. The preferable option is 1~100 mg/kg(avoirdupois)/day.

The pharmaceutical compositions include tablet, syrup, capsule, powder for injection, injection and inhalant.

The pharmaceutical compositions should be given by oral access, injection or inhalation through lung.

The preparation of Naringenin, Naringin and salts thereof is as follows:

Naringin can be synthesized according to the method by Rosenmund ( Rosenmund, Ber., 61, 2608( 1958 ) )and Zemlen, Bognar(Ber., 75, 648( 1942)). Naringin should be also obtained by extraction from botanic materials.

The botanic materials containing Naringin include Fruit of Citrus aurantium L., Fructus aurantii immaturus (Immature bitter orange), Citrus reticulata Blanco(Mandarin orange), Citrus paradisi Macf.(Grape pomelo), chrysoidine, Exocarpium citri grandis(Pummelo peel), Citrus sinensis (Linn.) Osbesk(Sweet orange) and so on.

The preparation of Naringin includes such steps: the botanic materials containing Naringin should be crushed first, then extracted by water for one to three times, then filtrate and combine the filtrate; the filtrate is concentrated and deposit in ethanol. The liquid is filtrated, then the filtrate is concentrated by eliminating ethanol firstly or directly separated by column chromatography with organic solvent. The organic solvent is eliminated from the eluent, then the rough Naringin is obtained. After one to ten times of recrystallization, monomer of Naringin is obtained.

There are three methods to prepare Naringenin:

Method 1: Naringenin should be obtained by the hydrolyzation of Naringin. The preparation of Naringenin includes such steps: Naringin is dissolved in water, stirred equably, hydrolysated in acid or alkali or enzyme. Then the liquid is filtrated and rough Naringenin is obtained from the deposit. After one to ten times of recrystallization, monomer of Naringenin is obtained.

The enzymes for hydrolyzation include glucuronidase and other enzymes that can break hexose.

Method 2: Naringenin should be also obtained by extraction from botanic materials containing Naringenin.

The botanic materials containing Naringenin include the buds of cherry and plum etc.

The preparation of Naringenin includes such steps: the botanic materials containing Naringenin should be crushed first, then extracted by organic solvent for one to three times, then filtrate, combine and concentrated the filtrate into extracts, crude Naringenin is obtained from the deposit. After one to ten times of recrystallization, monomer of Naringenin is obtained.

Method 3: Monomer of Naringenin should be obtained from the plant containing Naringenin by direct hydrolysation.

The preparation of Naringenin includes such steps: the botanic materials containing Naringenin should be crushed first, then extracted by water or organic solvent for one to three times, then filtrate and combine the filtrate into extracts; extracts should be dissolved by water or organic solvent, hydrolysised in acid or alkali or enzymes, then filtrated and the rough Naringenin is obtained from the deposit. After one to ten times recrystallization, monomer of Naringenin is obtained.

The enzymes for hydrolyzation include glucuronidase and other enzymes that can break hexose.

The organic solvent referred in the above methods includes alcohol, methanol, chloroform, ethyl acetate and petroleum.

The salt of Naringenin referred is metal salt which is synthesized with Naringenin and relevant alkali substance, its molecular formula is: $C_{15}H_{(12-n)}O_5\ Y_n$, n=1-3, Y is metal ion.

The preparation of the Naringenin salt includes such steps: Naringenin and relevant alkali substance react at 0~100°C, and create relevant metal salt of Naringenin; in the process of reaction, the mol ratio of Naringenin and the alkali substance is 1:1 to 1:3.

The alkali substance referred in above methods include sodium hydroxide, potassium hydroxide, potassium hydroxide, sodium bicarbonate, kalium bicarbonate, sodium carbonate, and potassium carbonate. The Naringenin salt synthesized is sodium or potassium salt of Naringenin, its molecular formula is: $C_{15}H_{(12-n)}O_5\ Y_n$, n=1-3, Y is sodium or potassium ion.

The salt of Naringin referred is metal salt is synthesized by Naringin and relevant alkali substance, which molecular formula is: $C_{27}H_{(32-n)}O_{14}\ Y_n$, n=1-2, Y is metal ion.

The Naringin salt is obtained by the following ways: Naringin and relevant alkali substance react at 0~40°C, and create metal salt of Naringin. In the process of reaction, the mol ratio of Naringin and the alkali substance is 1:1 ~ 1:2.

The alkali substance referred in the above methods includes sodium hydroxide, potassium hydroxide, potassium hydroxide, sodium bicarbonate, kalium bicarbonate, sodium carbonate, and potassium carbonate. The Naringin salt synthesized is sodium or potassium salt of Naringin, its molecular formula is: $C_{27}H_{(32-n)}O_{14}Y_n$, n=1-2, Y is sodium or potassium ion.

Another object of this invention is to provide the uses of Naringenin, Naringin and salts thereof in the treatment of cough and dispelling phlegm (mucus)

The treatment includes giving drug to the mammal such as the composition provided by the invention, especially to human beings.

Naringenin is a flavanones with three hydroxyl, the property of this compound is stable, it stay in original shape after entering into body; Naringin is a ramification of Naringenin, and it is a glycoside with that Naringenin combine a disaccharide at the seven site, then it is hydrolyzed because of the stomach acid, so it works as the form of Naringenin, while a little of Naringin can be exist as original shape because of incomplete hydrolyzation (Kazuo Ishii, Takashi Furuta, Yasuji Kasuya. Determination of Naringin and Naringenin in human plasma by high-performance liquid chromatography. Journal of Chromatography B, 683(1996);225-229). Naringenin salt changed into Naringenin because of stomach acid after entering into body, and Naringin salt is firstly transformed into Naringin and then hydrolyzed all because of stomach acid, into Naringenin to give an effect. The pharmacological experiments prove that the compositions of the invention have improving effects of treatment of cough and phlegmatical diseases, without side effects, and can be used to treat acute or chronic bronchitis, cold etc. relating cough and phlegmatical conditions. And they have the advantages of stable quality, small dose, rapid effect and so on.

The experiment of the treatment of cough on mouse by using Naringenin, Naringin and salt thereof indicates: the tolerance time of cough arisen by stimulation to the mouse are remarkably prolonged by Naringenin, Naringin and salt thereof, comparing to the blank control, and this remarkable difference is believable in statistics; when compared to hydrobromic acid dextromethorphan which is considered as a positive control drug, the tolerance time is prolonged and the curative effect is more remarkable with Naringenin, Naringin and salt thereof. That means: Naringenin, Naringin and salt thereof have improving effects of treatment of cough.

The experiment of the treatment of phlegmatical conditions on mouse by using Naringenin, Naringin and salt thereof indicates: the increases of phlegm in mouse's bronchia are remarkable enhanced by Naringenin, Naringin and salt thereof, comparing to the blank control, and this remarkable difference is believable in statistics; when compared with "Tankejin" which is considered as a positive control drug, the increases of phlegm in mouse's bronchia are remarkable enhanced and the curative effect is more remarkable with Naringenin, Naringin and salt thereof. That means: Naringenin, Naringin and salt thereof have improving effects of treatment of phlegm-related diseases.

The experiments of the invention prove that Naringenin, Naringin and salt thereof have improving effects of treatment of cough and phlegm-related diseases, without manifest toxicity or side effects in animal experiment of mouse. The animal experiments indicate that the animals hasn't toxic reaction when they were given the oral dose of 6g/kg Naringenin and salt thereof, although this dose is as 460 times as the normal dose for human beings; the animals haven't toxic reaction when they were given the oral dose of 3g/kg Naringin and salt thereof, although this dose is as 230 times as the normal dose for human beings.

To sum up, Naringenin, Naringin and salt thereof have improving effects of treatment of cough and phlegm-related diseases, without side effects, and can be used to treat acute or chronic bronchitis, cold etc. relating cough and phlegm-related conditions. So they can be use in preparation of drug for the treatment of cough and phlegm-related conditions.

The pharmaceutical compositions of Naringenin, Naringin and salt thereof have improving effects of treatment of cough and phlegm-related diseases, without side effects, and can be used to treat acute or chronic bronchitis, cold etc. relating cough and phlegm-related conditions. And they have the advantages of stable quality, small dose, rapid effect and so on.

## DETAILED DESCRIPTION OF THE INVENTION

A further illustration of the invention may be described with reference to the following examples.

The percents of solid in solid compound, liquid in liquid compound and solid in liquid compound are counted by wt/wt, vol/vol, wt/vol unless other explanation.

Example 1: Preparation of Monomer of Naringin

Raw material of fruit of Citrus aurantium L. should be chopped into decoction pieces, then submerged with 100°C water, then extracted by water for three times, one hour per time, then filtrate, combine and concentrate the filtrate into extracts, which should be passed through the chromatographic column of macroporous resin, washed down with water, and the chromatographic column should be washed down with 70% ethanol after abandoning the water, ethanol eluent should be gathered and concentrated into deposition. After three times of recrystallization with water, monomer of Naringin is obtained. Transfer ratio is 90.7%.

Example 2: Preparation of Monomer of Naringin

Raw material of Citrus reticulata Blanco (Mandarin orange) should be crushed, but not sieved, then submerged with 100°C water, then extracted from water for twice, one hour per time, then filtrate, combine and concentrate the filtrate, then absolute ethyl alcohol should be added till the ethanol concentration reaching 30%, after placed for a whole

night, then filtrated again, the supernate should be concentrated into extracts by eliminating ethanol. Extracts should be passed through the chromatographic column of polyamide, firstly washed down with water, and the chromatographic column should be washed down with 80% ethanol after abandoning the water, ethanol eluent should be gathered and concentrated into deposition. After five times of recrystallization with absolute alcohol, monomer of Naringin is obtained. Transfer ratio is 87.4%.

Example 3: Preparation of Monomer of Naringin

Raw material of Exocarpium citri grandis (Pummelo peel) should be should be chopped into decoction pieces, then submerged with 70°C water, then extracted from water for twice, three hours per time and get filtrated, the filtrate should be concentrated into extracts, which should be added with 95%ethanol till the ethanol concentration reaching 60%, after a whole night, some deposition appear, then the deposition should be filtrated and abandoned. Filtrate should be concentrated, and the liquid obtained should be passed through the chromatographic column of polyamide, washed down with water first, and the chromatographic column should be washed down with 80% ethanol after abandoning the water, ethanol eluent should be gathered and concentrated into deposition, then recrystallized with absolute ethyl alcohol for four times. Monomer of Naringin can be obtained. Transfer ratio is 89.7%.

Example 4: the preparation of Monomer of Naringin

Raw material of Citrus limon (Linn.) Burm. (lemon) needn't to be chopped, then extracted from water in ultrasonic condition for three times-one hour per time and get filtrated, the filtrate should be concentrated into extracts, then extracts should be direct passed through the chromatographic column of polyamide, washed down with water first, and the chromatographic column should be washed down with 95% ethanol after abandoning the water, ethanol eluent should be gathered, concentrated, and filtrated, then deposition obtained should be recrystallized with ethyl acetate for one time. Monomer of Naringin can be obtained. Transfer ratio is 85.3%.

Example 5: Preparation of Monomer of Naringin

Raw material of Fructus aurantii immaturus (Immature bitter orange) should be crushed and sieved with 20 mesh screen, then then extracted by water under the room temperature for three times-two days per time and get filtrated, the filtrate should be added with 95%ethanol till the ethanol concentration reaching 50%, then laid still before deposition appears. The deposition should be filtrated and abandoned. Filtrate should be concentrated, dissolved with water and passed through the chromatographic column of sephadex, washed down with water first, and the chromatographic column should be washed down with 40% ethanol after abandoning the water, ethanol eluent should be gathered , concentrated, and filtrated, then deposition obtained should be recrystallized with ethyl acetate for ten times. Monomer of Naringin can be obtained. Transfer ratio is 92.1 %.

Example 6: Preparation of Monomer of Naringin

Raw material of Citrus paradisi Macf. (Grape pomelo) should be chopped into decoction pieces, then be submerge with 100°C water, then extracted from water for two times-two hours per time, be filtrated, the filtrate should be concentrated into extracts, extracts should be passed through the chromatographic column of macroporous resin, washed down with water, and the chromatographic column should be washed down with 60% ethanol after abandoning the water, ethanol eluent should be gathered and concentrated into deposition, then the deposition should be recrystallized with water for three times. Monomer of Naringin can be obtained. Transfer ratio is 87.6%.

Example 7: Preparation of Monomer of Naringenin

Mixing up Naringin (purity: 98.2%) and water ten times than the Naringin, adjust pH to 1 with hydrochloric acid and mix them well. Heat up the mixture at 100°C for 2 hours to hydrolyze , filtrate, and the residue is crude Naringenin. Monomer of Naringenin is obtained by recrystallization with absolute ethanol for five times. The transfer ratio is 91.4%.

Example 8: Preparation of Monomer of Naringenin

Mixing up Naringin (purity: 98.2%) and water twenty times than the Naringin, adjust pH to 0 with sulphuric acid and mix them well. Heat up the mixture at 80°C for 3 hours to hydrolyze , filtrate, and the residue is crude Naringenin. Monomer of Naringenin is obtained by recrystallization with ethyl acetate for ten times. The transfer ratio is 89.3%.

Example 9: Preparation of Monomer of Naringenin

Mixing up Naringin (purity: 98.2%) and water fifty times than the Naringin, adjust pH to 13 with sodium hydroxide solution and mix them well. Heat up the mixture at 80°C for 1.5 hours to hydrolyze , adjust pH to 1 with hydrochloric acid and then filtrate. The residue is crude Naringenin. Monomer of Naringenin is obtained by recrystallization with acetone for one time. The transfer ratio is 86.9%.

Example 10: Preparation of Monomer of Naringenin

Mixing up Naringin ( purity : 98.2%) and water thirty-five times than the Naringin, put in glucuronidase till the enzyme concentration arise up to 0.1 mmol/L and mix them well. Heat up the mixture in a water bath at 80°C for 8 hours to hydrolyze , filtrate, and the residue is crude Naringenin. Monomer of Naringenin is obtained by recrystallization with absolute ethanol for four times. The transfer ratio is 94.8%.

Example 11: Preparation of Monomer of Naringenin

Abstract the flower buds of cherry three times with absolute ethanol which should overflow the raw materials, at 50°C and for 1 hour at every turn and then filtrate. Inspissate the filtrate to extracturm, deposit and filtrate. The residue is crude Naringenin. Monomer of Naringenin is obtained by recrystallization at a time with ethyl acetate. The transfer ratio is 90.7%.

Example 12: Preparation of Monomer of Naringenin

Abstract the flower buds of plum two times with acetone which should overflow the raw materials, at 25 °C and for 48 hours at every turn , filtrate and combine the filtrates. Inspissate the filtrates to extracturm, deposit over night to get sediments, dry the sediments to get crude Naringenin. Monomer of Naringenin is obtained by recrystallization five times with chloroform. The transfer ratio is 87.4%.

Example 13: Preparation of Monomer of Naringenin

Abstract the flower buds of plum one time with ethyl acetate which should overflow the raw materials, at 20°C and for 72 hours, and filtrate. Inspissate the filtrate to extracturm, deposit over night to get sediments, dry the sediments to get crude Naringenin. Monomer of Naringenin is obtained by recrystallization ten times with acetone. The transfer ratio is 85.6%.

Example 14: Preparation of Monomer of Naringenin

Crush the raw materials of Fructus aurantii immaturus (Immature bitter orange), sieved with a 20 mesh screen, abstract three times with water at room temperature, two days at every turn, filtrate and combine the filtrates. Inspissate the filtrates, adjust pH to 1 with hydrochloric acid, heat up the solution at 90°C to hydrolyze, filtrate to get sediments and dry the sediments to get crude Naringenin. Monomer of Naringenin is obtained by recrystallization four times with ethyl acetate. The transfer ratio is 90.7%.

Example 15: Preparation of Monomer of Naringenin

Cut the raw materials of Citrus paradisi Macf. (Grape pomelo) into decoction pieces, abstract at a time for 2 hours with 100°C water which should overflow the raw materials, filtrate and combine the filtrates. Inspissate the filtrates , adjust pH to 11 with sodium hydroxide solution, heat up at 70°C to hydrolyze, adjust pH to 1 with hydrochloric acid, filtrate to get sediments and dry the sediments to get crude Naringenin. Monomer of Naringenin is obtained by recrystallization three times with acetone. The transfer ratio is 89.2%.

Example 16: Preparation of Monomer of Naringenin

Abstract the raw material of Exocarpium citri grandis (Pummelo peel) (not crushed) three times with acetone, 24 hours at every turn, filtrate and combine the filtrates. Inspissate the solution into extracturm, dissolve with 30% ethanol , adjust pH to 2 with hydrochloric acid, heat up at 95 °C to hydrolyze, filtrate to get sediments and dry the sediments to get crude Naringenin. Monomer of Naringenin is obtained by recrystallization five times with chloroform. The transfer ratio is 87.7%.

Example 17: Preparation of Monomer of Naringenin

Cut the raw material of fruit of Citrus aurantium L. into decoction pieces, dip the pieces into 100°C water, adjust pH to 2.5 with hydrochloric acid, abstract three times for 1.5 hours at every turn, filtrate and combine the filtrates. Inspissate the solution into extracturm, deposit over night to get sediments. Separate the sediments and dissolve with acetone, filtrate, give up the residuum, collect all the solvent from the filtrates and dry the condensates to get crude Naringenin. Monomer of Naringenin is obtained by recrystallization three times with chloroform. The transfer ratio is 90.4%.

Example 18: Preparation of Monomer of Naringenin

Crush the raw material of Citrus grandus (L.) Osbrck var Shatmyn Hort, dip the raw materials into twelve times water (100°C), adjust pH to 14 with potassium hydroxide solution, abstract twice for 45 hours at every turn, filtrate and combine the filtrates. Inspissate the solution into extracturm, deposit over night to get sediments. Separate the sediments and dissolve with absolute ethanol, filtrate, give up the residuum, collect all the solvent from the filtrates and dry the condensates to get crude Naringenin. Monomer of Naringenin is obtained by recrystallization four times with acetone. The transfer ratio is 88.3%.

Example 19: Preparation of salt of Naringenin

Under temperature of 0°C, dissolve 1 mol Naringenins with ethanol and 1 mol sodium hydroxides with water, put the sodium hydroxide solution slowly into the Naringenin ethanol solution, mix around for 2 hours, and filtrate to get sodium salt of Naringenin.

In this example, sodium hydroxide can be replaced by equivalent potassium hydroxide or potassium bicarbonate or potassium carbonate to get potassium salt of Naringenin. Equivalent sodium bicarbonate or sodium carbonate can be also used as a substitute for sodium hydroxide to get sodium salt of Naringenin.

Example 20: Preparation of salt of Naringenin

Under temperature of 20°C, dissolve 1 mol Naringenins with ethanol and 3 mol sodium hydroxides with water, put the sodium hydroxide solution slowly into the Naringenin ethanol solution, mix around for 2 hours, and filtrate to get sodium salt of Naringenin.

In this example, sodium hydroxide can be replaced by equivalent potassium hydroxide or potassium bicarbonate or potassium carbonate to get potassium salt of Naringenin. Equivalent sodium bicarbonate or sodium carbonate can be also used as a substitute for sodium hydroxide to get sodium salt of Naringenin.

Example 21: Preparation of salt of Naringenin

Under temperature of 50°C, dissolve 1 mol Naringenins with ethanol and 2 mol sodium hydroxides with water, put the sodium hydroxide solution slowly into the Naringenin ethanol solution, mix around for 2 hours, and filtrate to get sodium salt of Naringenin.

In this example, sodium hydroxide can be replaced by equivalent potassium hydroxide or potassium bicarbonate or potassium carbonate to get potassium salt of Naringenin. Equivalent sodium bicarbonate or sodium carbonate can be also used as a substitute for sodium hydroxide to get sodium salt of Naringenin.

Example 22 : Preparation of salt of Naringenin

Under temperature of 100°C, dissolve 1 mol Naringenins with ethanol and 3 mol sodium hydroxides with water, put the sodium hydroxide solution slowly into the Naringenin ethanol solution, mix around for 2 hours, and filtrate to get sodium salt of Naringenin.

In this example, sodium hydroxide can be replaced by equivalent potassium hydroxide or potassium bicarbonate or potassium carbonate to get potassium salt of Naringenin. Equivalent sodium bicarbonate or sodium carbonate can be also used as a substitute for sodium hydroxide to get sodium salt of Naringenin.

Example 23: Preparation of salt of Naringin

Under temperature of 0°C, dissolve 1 mol Naringins with ethanol and 1 mol sodium hydroxides with water, put the sodium hydroxide solution slowly into the Naringin ethanol solution, mix around for 2 hours, and filtrate to get sodium

salt of Naringin.

In this example, sodium hydroxide can be replaced by equivalent potassium hydroxide or potassium bicarbonate or potassium carbonate to get potassium salt of Naringin. Equivalent sodium bicarbonate or sodium carbonate can be also used as a substitute for sodium hydroxide to get sodium salt of Naringin.

Example 24: Preparation of salt of Naringin

Under temperature of 20°C, 1mol Naringin was weighed accurately into a suitable flask with adequate ethanol adding to obtain solution I while 2 mol potassium hydroxide was weighed accurately with enough water adding to obtain solution II. After solution II was added slowly into solution I, with mixing round for 2 hours, and filtrate to get sodium salt of Naringin.

In this example, sodium hydroxide can be replaced by equivalent potassium hydroxide or potassium bicarbonate or potassium carbonate to get potassium salt of Naringenin. Equivalent sodium bicarbonate or sodium carbonate can be also used as a substitute for sodium hydroxide to get sodium salt of Naringenin.

Example 25: Preparation of salt of Naringin

Under temperature of 40°C, 1mol Naringin was weighed accurately into a suitable flask with adequate ethanol adding to obtain solution I while 2 mol potassium hydroxide was weighed accurately with enough water adding to obtain solution II. After solution II was added slowly into solution I, with mixing round for 2 hours, and filtrate to get sodium salt of Naringin.

In this example, sodium hydroxide can be replaced by equivalent potassium hydroxide or potassium bicarbonate or potassium carbonate to get potassium salt of Naringenin. Equivalent sodium bicarbonate or sodium carbonate can be also used as a substitute for sodium hydroxide to get sodium salt of Naringenin.

Example 26: The Purity Determination of monomer of Naringenin

Put adequate monomer of Naringenin which obtained by the method described above into a volumetric flask, then accurately adds methanol to the volume. After filtration by millipore filter of 0.45um, the solution was injected into the HPLC system and the contents of pure Naringenin in different sample were measured, with Naringenin offered by NICPBP as the reference substance. The results were shown in Table 1.

Chromatographic Conditions: Agilent1100 HPLC(Automatic sampler, deaerator, quaternary pump, column thermostat, PDA detector); Column: MARKER ODS (5μm, 4.0×250mm) ; Mobile Phase: Methanol-water(50:50); Detection Wavelength: 288nm; Column Temperature: 30°C; Flow Velocity: 1ml/min; Injection Volume: 5μl.

Table 1 Results for Purity Test of Sample of Naringenin

| Sample | Concentration of Sample (mg/ml) | Injection Volume (μl) | Peak Area (mAU) | Concentration of Naringenin (mg/ml) | Content of Naringenin (%) |
|---|---|---|---|---|---|
| Contrast | 0.500 | 5 | 3145.74885 | 0.500 | 100 |
| NO.1 | 0.462 | 5 | 2850.04845 | 0.453 | 98.1 |
| NO.2 | 0.472 | 5 | 2925.54643 | 0.465 | 98.5 |
| NO.3 | 0.521 | 5 | 3189.78933 | 0.507 | 97.4 |
| NO.4 | 0.483 | 5 | 2925.54649 | 0.465 | 96.3 |
| NO.5 | 0.512 | 5 | 3189.78934 | 0.507 | 99.0 |
| NO.6 | 0.502 | 5 | 3019.91890 | 0.480 | 95.6 |
| NO.7 | 0.439 | 5 | 2661.30353 | 0.423 | 96.3 |
| NO.8 | 0.494 | 5 | 3032.50189 | 0.482 | 97.5 |
| NO.9 | 0.508 | 5 | 3095.41687 | 0.492 | 96.9 |
| NO.10 | 0.517 | 5 | 3196.08083 | 0.508 | 98.3 |
| NO.11 | 0.486 | 5 | 2988.46141 | 0.475 | 97.7 |

As it shows in Table 1, the purity of monomer of Naringenin in the extracted samples was above 95%. It meant that the purity of samples which were extracted by the method shown in this invention was very high.

Example 27: The Purity Determination of monomer of Naringin

Put adequate monomer of Naringin which obtained by the method described above into a volumetric flask, and then accurately add methanol to the volume. After filtration by millipore filter of 0.45um, the solution was injected into the HPLC system and the contents of pure Naringin in different samples were measured, with Naringin offered by NICPBP as the reference substance. The results were shown in Table 2.

Chromatographic Conditions: Agilent1100 HPLC(Automatic sampler, deaerator, quaternary pump, column thermostat, PDA detector); Column: MARKER ODS (5μm, 4.0×250mm) ; Mobile Phase: ACN-0.05M KH2PO4 Buffer (20: 80) ; Detection Wavelength: 283nm; Column Temperature: 30°C; Flow Velocity: 1ml/min; Injection Volume: 5μl.

Table 2 Results for Purity Test of Sample of Naringin

| Sample | Concentration of Sample (mg/ml) | Injection Volume (μl) | Peak Area (mAU) | Concentration of Naringin (mg/ml) | Content of Naringin (%) |
|---|---|---|---|---|---|
| contrast | 0.400 | 5 | 2743.35889 | 0.400 | 100 |
| NO.1 | 0.396 | 5 | 2626.76614 | 0.383 | 96.7 |
| NO.2 | 0.424 | 5 | 2853.09325 | 0.416 | 98.2 |
| NO.3 | 0.385 | 5 | 2571.89896 | 0.375 | 97.4 |
| NO.4 | 0.417 | 5 | 2798.22607 | 0.408 | 97.9 |

In conclusion, it meant that the extracting methods described above could efficiently extract the monomer of Naringin and Naringenin from the relevant plant materials, and also successfully obtain monomer of Naringenin by hydrolyzing Naringin. In addition to this, the results shown that purity level of both Naringin and Naringenin was above 95%. The extracting technology was simple and feasible.

Example 28: Pharmacology Test of Naringin for the treatment of cough

1.Animals: As the stock animals, totally 130 NIH mice, male, each weighed 18.2~21.7g within ordinary standard. Weigh and number each mice accurately first, then pick out the 120 healthy ones who weighed in range from 18.5~21.0g. Ranked in weigh, they were divided into 8 groups in random with each 15. Set up reference groups of negative and positive, and sample groups of high-dose, mid-dose, low-dose Naringin and sodium Naringinate.

2. Source and Preparation of the Samples:

1) Blank control group: physiological saline, 0.9% NaCl;

2) Positive control group: 30 mg dextromethorphan hydrobromide was weighed into a 20ml volumetric flask, and physiological saline was accurately added to the volume to obtain the solution for positive control at concentration of 5mg/ml.

3) Group of low-dose sample Naringin: Proper amount of Naringin was weighed into a suitable volumetric flask, and physiological saline was added to the volume to obtain the solution at concentration of 0.5mg/ml.

4) Group of mid-dose sample Naringin: Proper amount of Naringin was weighed into a suitable volumetric flask, and physiological saline was added to the volume to obtain the solution at concentration of 1.5mg/ml.

5) Group of high-dose sample Naringin: Proper amount of Naringin was weighed into a suitable volumetric flask, and physiological saline was added to the volume to obtain the solution at concentration of 4.5mg/ml.

6) Group of low-dose sample sodium Naringinate: Proper amount of sodium Naringin was weighed into a suitable volumetric flask, and physiological saline was added to the volume to obtain the solution at concentration of 0.5mg/ml.

7) Group of mid-dose sample sodium Naringinate: Proper amount of sodium Naringin was weighed into a suitable volumetric flask, and physiological saline was added to the volume to obtain the solution at concentration of 1.5mg/ml.

8) Group of high-dose sample sodium Naringinate: Proper amount of sodium Naringin was weighed into a

suitable volumetric flask, and physiological saline was added to the volume to obtain the solution at concentration of4.5mg/ml.

Naringin needed in this Example was obtained by the extracting method above, with its purity above 95%.

3. Experimental Method( Spraying with concentrated ammonia liquor) :

One hour after intra-gastrointestinal administering for the mice, spray with concentrated ammonia liquor for certain intervals. Immediately the spraying was over, put away the mice outside and observe the tussive action. Mark down the times for which they coughed within 1 minute. Only when they coughed, which was characteristic of compression of abdomen or chest with their mouths open, and making coughing noise, for more than 3 times within a minute, tussive action could be admitted.

4. Experimental Procedure:

Calculate the median effective dose($EDT_{50}$) with sequential trials. Work out the amount of R with the formula below. If R is greater than 130%, it means the drug will be effective on antitussive action. If R is greater than 150%, it means the drug will be remarkably effective on antitussive action. The formula is shown as below:

$$R = \frac{EDT_{50} \text{ of the treatmnet}}{EDT_{50} \text{ of the control}} \times 100\%$$

$EDT_{50}$=log-1c/n(n for amount of animals, c for summation of rx, r for amount of animals in each group, x for logarithm of the dose (time for spraying))

5.Result:

According to statistical data of all the samples, their $EDT_{50}$ and the effect of relieving a cough were shown in Table 3.

Table 3 Results of All the Samples For the Treatment of Cough

| Groups | | Dose (ml/20g) | Concentration (mg/ml) | $EDT_{50}$ (sec.) | R Value (%) | Effect of relieving cough |
|---|---|---|---|---|---|---|
| NO. | Samples | | | | | |
| 1 | Physiological Saline | 0.2 | 0 | 40.52 | - | - |
| 2 | Dextromethorphan Hydrobromide | 0.2 | 1.5 | 56.82 | 140.23 | effective |
| 3 | low-dose sample naringin | 0.2 | 0.5 | 56.41 | 139.21 | effective |
| 4 | mid-dose sample naringin | 0.2 | 1.5 | 61.33 | 151.36 | remarkably effective |
| 5 | high-dose sample naringin | 0.2 | 4.5 | 65.53 | 161.72 | remarkably effective |
| 6 | low-dose sample sodium naringinate | 0.2 | 0.5 | 57.68 | 142.35 | effective |
| 7 | mid-dose sample sodium naringinate | 0.2 | 1.5 | 63.15 | 155.84 | remarkably effective |
| 8 | high-dose sample sodium naringinate | 0.2 | 4.5 | 66.43 | 163.94 | remarkably effective |

Compared with resistance time against cough induced by intra-gastrointestinal administering of the reference substance dextromethorphan hydrobromide, the time of the Naringin samples was remarkably longer. It was statistically significant, which came to a conclusion that Naringin was remarkably effective on antitussive action.

Example 29: Pharmacological test of Naringin for the treatment of phlegm-related conditions

1. Animal: 80 healthy female mice weighing 19.1-22.3g was grouped into 8 groups random after labeled, which was selected from 90 female mice weighing 18.8-22.7g. Every group was 10 animals. Eight group were set including negative control group, positive control group, high dose group of Naringin, middle dose group of Naringin, low dose group of Naringin, high dose group of sodium salt of Naringin, middle dose group of sodium salt of Naringin, low dose group of sodium salt of Naringin. Mice were oral administered with a dose of 10 mg/kg.
2. Sample source and preparation

   1) Blank control group: physiological saline, 0.9% NaCl;
   2) Positive control group: 0.2g Tankejing was dissolve into 10 ml physiological saline and the sample concentration was 20 mg/ml to obtain the solution for positive control.
   3) low dose group of Naringin: 0.5 mg/ml solution of Naringin was prepared by weighting out the appropriate amount of 5 mg Naringin and dissolving it by ultrasonication in 10 ml physiological saline.
   4) middle dose group of Naringin: 1.5 mg/ml solution of Naringin was prepared by weighting out the appropriate amount of 15 mg Naringin and dissolving it by ultrasonication in 10 ml physiological saline.
   5) high dose group of Naringin: 4.5 mg/ml solution of Naringin was prepared by weighting out the appropriate amount of 45 mg Naringin and dissolving it by ultrasonication in 10 ml physiological saline.
   6) low dose group of sodium salt of Naringin: 0.5 mg/ml solution of sodium salt of Naringin was prepared by weighting out the appropriate amount of 5 mg sodium salt of Naringin and dissolving it by ultrasonication in 10 ml physiological saline.
   7) middle dose group of sodium salt of Naringin: 1.5 mg/ml solution of sodium salt of Naringin was prepared by weighting out the appropriate amount of 15 mg sodium salt of Naringin and dissolving it by ultrasonication in 10 ml physiological saline.
   8) high dose group of sodium salt of Naringin: 4.5 mg/ml solution of sodium salt of Naringin was prepared by weighting out the appropriate amount of 45 mg sodium salt of Naringin and dissolving it by ultrasonication in 10 ml physiological saline.

3. Experimental Method:

   1) preparations of Phenol Red calibration standard: 12.5 ng/ml solution of Phenol Red was prepared by weighting out the appropriate amount of Phenol Red and dissolving it by ultrasonication in 5% $NaHCO_3$. The series solution including 6.25 ng/ml, 3.125 ng/ml, 1.5625 ng/ml, 0.7813 ng/ml, 0.3906 ng/ml, 0.1953 ng/ml, 0.0977 ng/ml, 0.0488 ng/ml was prepared by diluting, which was detected by ultraviolet spectrometer. The calibration curve was calculated by concentration of Phenol Red as y-axis and OD value as x-axis. And the excretion account of Phenol Red in mice was quantitated by calibration curve.
   2) Animals were fasted for 12 hr before dosing.
   3) Animals were oral administered with labeled number, each animal was administered with 3 min and each group was accomplished in 30 min.
   4) After administered 30 min, every animal was injected 2 ml 5% Phenol Red solution via celiac injection. The time of every animal was 3 min.
   5): Every animal was executed with 3 min interval after celiac injection 30 min, and animal trachea was peeled off with forceps after neck skin sniped, which was riveted on operating table.
   6): plasma and Phenol Red adhered to trachea ektexine was sweeped by 0.9% NaCl solution with an injector. And filter paper was used to blot up the solution.
   7): Scissors cut down the animal trachea including thyroid cartilage and trachea back-end.
   8): The trachea was set in test tube with 1.5 ml 5% $NaHCO_3$.
   9): The experiment of every animal was completed in 3 min. And the method was same to every animal.
   10): The Phenol Red of trachea was released in test tube by ultrasonication 5 min.
   11): OD value of solution in test tube was detected by 721 ultraviolet spectrometer at 546nm.
   12): The trachea was dip in test tube for 24 hr and OD value of solution in test tube was detected again after 24 hr.
   13): After that, OD value of Phenol Red in mice was quantitated by calibration curve. The calibration curve is: Y=7.6266X-0.0554. X indicates OD value; Y indicates excretion account of Phenol Red.
   14): Rectified excretion account of Phenol Red was calculated by account of Phenol Red and weight of animal. Statistical significance of the data was determined by Student's t-test with SPSS 8.0. Rectified excretion account of Phenol Red= Account of Phenol Red (ng) / weight of animal (kg).

4. Result

According to statistical data of all the samples, the excretion quantity of Phenol Red was show in Table 4.

Table 4 Effect of Naringin for the Treatment of Phlegm-related Conditions (X±S)

| Groups | | Dose (mg/kg) | Excretion quantity of Phenol Red (ng) | Rectified excretion quantity of Phenol Red[a] (ng/kg) | P value | Dispel phlegm ratio[b] (%) |
|---|---|---|---|---|---|---|
| NO. | Sample | | | | | |
| 1 | Physiological Saline | 0 | 0.3031±0.0747 | 15.4576±4.0701 | - | - |
| 2 | Tankejing | 200 | 0.8621±0.3981 | 45.0285±21.8238 | P< 0.01 | 291.30 |
| 3 | Low dose group of Naringin | 5 | 0.4914±0.2215 | 25.5811±11.8338 | 0.05 | 159.02 |
| 4 | Middle dose group of Naringin | 15 | 0.5296±0.2195 | 26.7597±13.1502 | P< 0.01 | 173.12 |
| 5 | High dose group of Naringin | 45 | 1.1488±0.2431 | 64.4162±18.7239 | P< 0.01 | 416.72 |
| 6 | Low dose group of sodium salt of Naringin | 5 | 0.5250±0.1265 | 26.1805±6.9069 | P< 0.05 | 169.37 |
| 7 | Middle dose group of sodium salt of Naringin | 15 | 0.5748±0.1417 | 29.3153±7.7189 | P< 0.01 | 189.65 |
| 8 | High dose group of sodium salt of Naringin | 45 | 1.3115±0.3232 | 66.8867±17.6117 | P< 0.01 | 432.71 |

a: Rectified excretion quantity of Phenol Red= Excretion quantity of Phenol Red (ng) / weight of animal (kg).
b: Dispel phlegm ratio= Excretion quantity of Phenol Red of the group given drug / Phenol Red account of blank control group × 100%
Excretion quantity of Phenol Red (ng)= OD value×7.6266-0.0554

Compared the exudates increase of mice trachea after oral administered Naringin and Tankejing, three dose of Naringin, including low, middle and high, were statistical significance stimulate the exudates increase of mice trachea, which was statistical different with negative control group. The high dose group of Naringin was statistical significance stimulate the exudates increase of mice trachea compared to the positive control group of Tankejing, indicated that the dispel phlegm effect of Naringin are statistical better than Tankejing. The result indicates that Naringin has a wonderful dispel phlegm effect.

Example 30: Relieve a cough Pharmacology of Naringenin

1. Animal: 120 healthy male mice weighing 19.5-22.1g was grouped into 8 groups random after labeled, which was selected from 130 male mice weighing 19.3-22.6g. Every group was 15 mice. Eight group were set including negative control group, positive control group, high dose group of Naringenin, middle dose group of Naringenin, low dose group of Naringenin, high dose group of sodium salt of Naringenin, middle dose group of sodium salt of Naringenin, low dose group of sodium salt of Naringenin.
2. Sample source and preparation:

1) Blank control group: physiological saline, 0.9% NaCl;
2) Positive control group: 30mg Dextromethorphan Hydrobromide was dissolve into 20 ml physiological saline and the sample concentration was 1.5 mg/ml to obtain the solution for positive control.
3) low dose group of Naringenin: weighting out the appropriate amount of Naringenin and dissolving it in physiological saline, then the sample concentration was 0.5 mg/ml.

4) middle dose group of Naringenin: weighting out the appropriate amount of Naringenin and dissolving it in physiological saline, then the sample concentration was 1.5mg/ml.

5) high dose group of Naringenin: weighting out the appropriate amount of Naringenin and dissolving it in physiological saline, then the sample concentration was 4.5 mg/ml.

6) low dose group of sodium salt of Naringenin: weighting out the appropriate amount of sodium salt of Naringenin and dissolving it in physiological saline, then the sample concentration was 0.5 mg/ml.

7) middle dose group of sodium salt of Naringenin: weighting out the appropriate amount of sodium salt of Naringenin and dissolving it in physiological saline, then the sample concentration was 1.5 mg/ml.

8) high dose group of sodium salt of Naringenin: weighting out the appropriate amount of sodium salt of Naringenin and dissolving it in physiological saline, then the sample concentration was 4.5 mg/ml.

3. Experimental method of spray by high concentration ammonia

Animals were oral administered, and were sprayed one hour later. After the spray took out the animals and observed the tussive action. Observed the time of tussis in one minutes, if it appeared typical tussis three times in one minutes (shrinking the abdomen muscle or shrinking chest, puffing mouth at the same time, sometimes appearing tussive sound), we consider it "appearing tussis", otherwise "no tussis".

4. Process of the experiment

We calculated the spraying time that half of mice were tusisive by sequential method. And calculated the R, the drug was provided with effect of relieve a cough if R>130%. The drug was provided with remarkable effect of relieve a cough if R>150%. Underside is expressions:

$$R = \frac{EDT_{50} \text{ of the treatment}}{EDT_{50} \text{ of the control}} \times 100\%$$

$EDT_{50}=\log^{-1}c/n$ ('n' is the number of animal, 'c' is the sum of 'rx' value, 'r' is the number of animal in every group, 'x' is the logarithm of dose(namely spray time).)

5. Result

According to statistical data of all the samples, their $EDT_{50}$ and the effect of relieving a cough were shown in Table 5.

Table 5 Effect of relieving cough in each group ($X \pm S$)

| Groups | | Dose (mg/20g) | Sample concentration (mg/ml) | $EDT_{50}$ (sec.) | R Value (%) | Effect of relieving cough |
|---|---|---|---|---|---|---|
| NO. | Sample | | | | | |
| 1 | Physiological Saline | 0.2 | 0 | 38.9 | - | - |
| 2 | Dextromethorphan Hydrobromide | 0.2 | 1.5 | 53.70 | 138.05 | effect |
| 3 | Low dose group of naringenin | 0.2 | 0.5 | 51.29 | 131.85 | effect |
| 4 | Middle dose group of naringenin | 0.2 | 1.5 | 66.07 | 169.85 | remarkably effect |
| 5 | High dose group of naringenin | 0.2 | 4.5 | 66.61 | 171.23 | remarkably |
| 6 | Low dose group of sodium salt of naringenin | 0.2 | 0.5 | 55.35 | 142.3 | effect |

(continued)

| Groups | | Dose (mg/20g) | Sample concentration (mg/ml) | $EDT_{50}$ (sec.) | R Value (%) | Effect of relieving cough |
|---|---|---|---|---|---|---|
| NO. | Sample | | | | | |
| 7 | Middle dose group of sodium salt of naringenin | 0.2 | 1.5 | 69.48 | 178.6 | remarkably effect |
| 8 | High dose group of sodium salt of naringenin | 0.2 | 4.5 | 73.56 | 189.1 | remarkably effect |

Compared the time of appearing tussis after oral administered Naringenin and Dextromethorphan Hydrobromide, Naringenin was statistical significance prolonging the time, which was statistical different with positive control group. Compared the time of appearing tussis after oral administered sodium salt of Naringenin and Dextromethorphan Hydrobromide, sodium salt of Naringenin was statistical significance prolonging the time, which was statistical different with positive control group. The result indicates that Naringenin and sodium salt of Naringenin has a wonderful relieve a cough effect.

Example 31: Dispel phlegm Pharmacology of Naringenin

1. Animal: 80 healthy female mice weighing 18.5-22.0g was grouped into 8 groups random after labeled, which was selected from 90 female mice weighing 18.0-22.1g. Every group was 10 mice. Eight groups were set including negative control group, positive control group, high dose group of Naringenin, middle dose group of Naringenin, low dose group of Naringenin, high dose group of sodium salt of Naringenin, middle dose group of sodium salt of Naringenin, low dose group of sodium salt of Naringenin. Mice were oral administered with a dose of 10 mg/kg.
2. Sample source and preparation

    1) Blank control group: physiological saline, 0.9% NaCl;
    2) Positive control group: 0.2g Tankejing was dissolve into 10 ml 0.9% NaCl and the sample concentration was 20 mg/ml to obtain the solution for positive control.
    3) low dose group of Naringenin: 0.5 mg/ml solution of Naringenin was prepared by weighting out the appropriate amount of 5 mg Naringenin and dissolving it by ultrasonication in 10 ml physiological saline.
    4) middle dose group of Naringenin: 1.5 mg/ml solution of Naringenin was prepared by weighting out the appropriate amount of 15 mg Naringenin and dissolving it by ultrasonication in 10 ml physiological saline.
    5) high dose group of Naringenin: 4.5 mg/ml solution of Naringenin was prepared by weighting out the appropriate amount of 45 mg Naringenin and dissolving it by ultrasonication in 10 ml physiological saline.
    6) low dose group of sodium salt of Naringenin: 0.5 mg/ml solution of sodium salt of Naringenin was prepared by weighting out the appropriate amount of 5 mg sodium salt of Naringenin and dissolving it by ultrasonication in 10 ml physiological saline.
    7) middle dose group of sodium salt of Naringenin: 1.5 mg/ml solution of sodium salt of Naringenin was prepared by weighting out the appropriate amount of 15 mg sodium salt of Naringenin and dissolving it by ultrasonication in 10 ml physiological saline.
    8) high dose group of sodium salt of Naringenin: 4.5 mg/ml solution of sodium salt of Naringenin was prepared by weighting out the appropriate amount of 45 mg sodium salt of Naringenin and dissolving it by ultrasonication in 10 ml physiological saline.

3. Experimental Method:

    1) preparations of Phenol Red calibration standard: 12.5 ng/ml solution of Phenol Red was prepared by weighting out the appropriate amount of Phenol Red and dissolving it by ultrasonication in 5% $NaHCO_3$. The series solution including 6.25ng/ml, 3.125ng/ml, 1.5625 ng/ml, 0.7813 ng/ml, 0.3906 ng/ml, 0.1953 ng/ml, 0.0977 ng/ml, 0.0488 ng/ml was prepared by diluting, which was detected by ultraviolet spectrometer. The calibration curve was calculated by concentration of Phenol Red as y-axis and OD value as x-axis. And the excretion account of Phenol Red in mice was quantitated by calibration curve.

2) Animals were fasted for 12 hr before dosing.
3) Animals were oral administered with labeled number, each animal was administered with 3 min and each group was accomplished in 30 min.
4) After administered 30 min, every animal was injected 2 ml 5% Phenol Red solution via celiac injection. The time of every animal was 3 min.
5) Every animal was executed with 3 min interval after celiac injection 30 min, and animal trachea was peeled off with forceps after neck skin sniped, which was riveted on operating table.
6) plasma and Phenol Red adhered to trachea ektexine was sweeped by 0.9% NaCl solution with an injector. And filter paper was used to blot up the solution.
7) Scissors cut down the animal trachea including thyroid cartilage and trachea back-end.
8): The trachea was set in test tube with 1.5 ml 5% $NaHCO_3$.
9) The experiment of every animal was completed in 3 min. And the method was same to every animal.
10) The Phenol Red of trachea was released in test tube by ultrasonication 5 min.
11) OD value of solution in test tube was detected by 721 ultraviolet spectrometer at 546nm.
12) The trachea was dip in test tube for 24 hr and OD value of solution in test tube was detected again after 24 hr.
13) After that, OD value of Phenol Red in mice was quantitated by calibration curve. The calibration curve is: Y=7.6266X-0.0554. X indicates OD value; Y indicates excretion account of Phenol Red.
14) Rectified excretion account of Phenol Red was calculated by account of Phenol Red and weight of animal. Statistical significance of the data was determined by Student's t-test with SPSS 8.0. Rectified excretion account of Phenol Red= Account of Phenol Red (ng) / weight of animal (kg)

4. Result

According to statistical data of all the samples, the excretion quantity of Phenol Red was show in Table 6.

Table 6 Effect of Naringenin and Salts Thereof for the Treatment of Phleg-related Conditions (X±S)

| Groups | | Dose (mg/kg) | Excretion quantity of Phenol Red(ng) | Rectified excretion quantity of Phenol Red[a] (ng/kg) | P value | Dispel phlegm ratio[b] (%) |
|---|---|---|---|---|---|---|
| NO. | Sample | | | | | |
| 1 | Physiological Saline | 0 | 0.9385±0.4148 | 46.7282±22.6106 | - | - |
| 2 | Tankejing | 200 | 1.3343±0.7932 | 70.3811±43.6970 | P<0.05 | 150.62 |
| 3 | Low dose group of Naringenin | 5 | 1.2757±0.4020 | 68.2832±23.6102 | P<0.05 | 146.13 |
| 4 | Middle dose group of Naringenin | 15 | 1.3523±0.9303 | 74.7504±57.2020 | P<0.05 | 159.97 |
| 5 | High dose group of Naringenin | 45 | 1.9257±0.6713 | 102.7325±41.0313 | P<0.01 | 219.85 |
| 6 | Low dose group of sodium salt of Naringenin | 5 | 1.3029±0.3069 | 69.4942±16.3724 | P<0.05 | 148.72 |
| 7 | Middle dose group of sodium salt of Naringenin | 15 | 1.4225±0.7361 | 75.8773±9.2651 | P<0.05 | 162.38 |
| 8 | High dose group of sodium salt of Naringenin | 45 | 1.9705±0.6898 | 105.1057±6.7945 | P<0.01 | 224.93 |
| a: Rectified excretion quantity of Phenol Red= Excretion quantity of Phenol Red (ng) / weight of animal (kg).<br>b: Dispel phlegm ratio= Excretion quantity of Phenol Red of the group given drug / Phenol Red account of blank control group × 100% | | | | | | |

$$\text{Excretion quantity of Phenol Red (ng)} = \text{OD value} \times 7.6266 - 0.0554$$

Compared the exudates increase of mice trachea after oral administered Naringenin and Tankejing, three dose of Naringenin, including low, middle and high, were statistical significance stimulate the exudates increase of mice trachea, which was statistical different with negative control group. The high dose group of Naringenin was statistical significance stimulate the exudates increase of mice trachea compared to the positive control group of Tankejing, indicated that the dispel phlegm effect of Naringenin are statistical better than Tankejing. Compared the exudates increase of mice trachea after oral administered sodium salt of Naringenin and Tankejing, three dose of sodium salt of Naringenin, including low, middle and high, were statistical significance stimulate the exudates increase of mice trachea, which was statistical different with negative control group. The high dose group of sodium salt of Naringenin was statistical significance stimulate the exudates increase of mice trachea compared to the positive control group of Tankejing, indicated that the dispel phlegm effect of sodium salt of Naringenin are statistical better than Tankejing.The result indicates that Naringenin and sodium salt of Naringenin have a wonderful dispel phlegm effect.

Example 32: Toxicological experiment of Naringin

With temperature in the range of 24±1°C and humidity in the range of 65±5 %,choose 20 NIH little mice which are 7-8 years old ,healthy and clean, male and female for each half ,their weights are between 20 and 22 gram.. Antisepsis the feed and water, before and in the layoff period of the test, all the mice should be feed under the normal feeding condition.

Dissolve Naringin in 0.5 % Tween80, the concentration is 300mg/ml,this liquor is given to little mice by oral access, the dosage is 0.2ml/20g weight of little mice. After administration ,they are observed for 1,4,8,12 hours, and then once per 12 hours. Observe the deaths of little mice ,and record the changes in little mice's weight as well as other symptoms. In the tenth day, kill little mice by breaking their necks, and take each apparatus for pathological inspection.

In the tenth day, all little mice are still alive .toxic reaction can't be found under the dosage of 3.0g/kg. This dosage is as many as 230 the normal dosage. Every apparatus of little mice is normal , pathological changes have not been found, and the weights of little mice are not found lightening in ten days. Therefore, this invention of Naringin medicine doesn't have toxic reaction when given by oral access.

Example 33: Toxicological experiment of sodium salt of Naringin

With temperature in the range of 25±1 °C and humidity in the range of 75±5 %,choose 20 NIH little mice which are 7-8 years old, healthy and clean, male and female for each half , their weights are between 18.5 and 20.5 gram.. Antisepsis the feed and water, before and in the layoff period of the test, all the mice should be feed under the normal feeding condition.

Dissolve Naringin sodium salt medicine in brine, the concentration is 300mg/ml, this liquor is given to little mice by oral access, the dosage is 0.2ml/20g weight of little mice. After administration ,they are observed for 1,4,8,12 hours, and then once per 12 hours. Observe the deaths of little mice, and record the changes in little mice's weights as well as other symptoms. In the tenth day, kill little mice by breaking their necks, and take each apparatus for pathological inspection..

In the tenth day, all little mice are still alive, toxic reaction can't be found under the dosage of 3.0g/kg. This dosage is as many as 230 the normal dosage. Every apparatus of little mice is normal , pathological changes have not been found, and the weights of little mice are not found lightening in ten days. Therefore, this invention of Naringin sodium salt medicine doesn't have toxic reaction when given by oral access.

Example 34: Toxicological experiment of Naringenin

With temperature in the range of 28±1°C and humidity in the range of 70±5%, choose 20 NIH little mice which are 7-8 years old ,healthy and clean, male and female for each half, their weights are between 19 and 21 gram. Antisepsis the feed and water, before and in the layoff period of the test, all the mice should be feed under the normal feeding condition.

Dissolve Naringenin in 0.5% Tween80, the concentration is 300mg/ml, this liquor is given to little mice by oral access, the dosage is 0.4ml/20g weight of little mice. After administration, they were observed for 1,4,8,12 hours, and then once per 12 hours. Observe the deaths of little mice, and records the changes in little mice's weight as well as other symptoms. In the tenth day, kill little mice by breaking their necks, and take each apparatus for pathological inspection.

In the tenth day, all little mice are still alive, toxic reaction can't be found under the dosage of 6.0g/kg. This dosage is as many as 460 the normal dosage. Every apparatus of little mice is normal, pathological changes have not

been found, and the weights of little mice are not found lightening in ten days. Therefore, this invention of Naringenin medicine doesn't have toxic reaction when given by oral access.

Example 35: Toxicological experiment of sodium salt of Naringenin

With temperature in the range of 24±1°C and humidity in the range of 65±5%, choose 20 NIH little mice which are 7-8 years old ,healthy and clean, male and female for each half ,their weights are between 18.2 and 22.5 gram.. Antisepsis the feed and water, before and in the layoff period of the test, all the mice should be feed under the normal feeding condition.

Dissolve Naringenin sodium salt medicine in brine, the concentration is 300mg/ml, this liquor is given to the little mice by oral access, the dosage is 0.4ml/20g weight of little mice. After administration, they were observed for 1,4,8,12 hours, and then once per 12 hours. Observe the deaths of little mice ,and record the changes in little mice's weight as well as other symptoms. In the tenth day, kill little mice by breaking their necks, and take each apparatus for pathological inspection.

In the tenth day, all the little mice are still alive, toxic reaction can't be found under the dosage of 6.0g/kg. This dosage is as many as 460 the normal dosage. Every apparatus of little mice is normal , pathological changes have not been found , and the weights of little mice are not found lightening in ten days. Therefore, this invention of Naringenin sodium salt medicine doesn't have toxic reaction when given by oral access.

Example 36: Capsule of Naringin

The preparation of the capsule of Naringin is as follows:

| | |
|---|---|
| Naringin | 100g |
| dry starch | 40g |
| miropowder of silica gel | 10g |

Mix above supplementary materials and Naringin equably, the mixture is filled into transparent capsule, so the capsule of Naringin is given. Filling level: 150mg/capsule.

Example 37: Tablet of Naringin

The preparation of the tablet of Naringin is as follows:

| | |
|---|---|
| Naringin | 750.0g |
| starch | 722.5g |
| starch slurry (14%) | 25.0g |
| magnesium stearate | 2.5g |
| total | 1500.0g |

Mix Naringin and starch equably, add the starch slurry and continue to stirred into the damp mass, then sieved with the 10 mesh nylon screen to make grains, ventilate to dry under 80-90°C, magnesium stearate is added to dry grains, then the grains pass the 12 mesh nylon screen, mixed equably, and pressed to tablet. There are 5000 pieces totally, and each piece weighs about 0.3 g.

Example 38: Capsule ofNaringenin

The preparation of the capsule of Naringenin is as follows:

| | |
|---|---|
| Naringenin | 60g |
| dry starch | 35g |
| miropowder of silica gel | 5g |

Mix above supplementary materials and Naringin equably, the mixture is filled into transparent capsule, so the capsule of Naringenin is given. Filling level: 150mg/capsule.

Example 39: Tablet ofNaringenin

The preparation of the tablet of Naringenin is as follows:

| | |
|---|---|
| Naringenin | 500.0g |
| starch | 472.5g |
| starch slurry (14%) | 25.0g |
| magnesium stearate | 2.5g |
| total | 1000.0g |

Mix Naringenin and starch equably, add the starch slurry and continue to stirred into the damp mass, and sieved with the 10 mesh nylon screen to to make grains, ventilate to dry under 80-90°C, magnesium stearate is added to dry grains, then the grains pass the 12 mesh nylon screen, mixed equably, and pressed to make tablet. There are 10000 pieces totally, and each piece weighs about 0.1 g.

Example 40: Inhalant of Naringenin

The preparation of the inhalant of Naringenin is as follows:

| | |
|---|---|
| Naringenin | 100g |
| lactose | 500g |
| poloxamer | 1g |
| miropowder silica gel | 10g |
| L-leucine | 0.5g |
| PEG400(50%) aqueous solution | 300g |
| Total | 911.5g |

Dissolve Naringenin, lactose, poloxamer and L-leucine with PEG400(50%) aqueous solution, then spray to dry, miropowder silica gel is added to the dry powder, mixing equably, finally the powder is filled into inhale equipment.

Example 41: Injection of sodium salt of Naringin

Take 100g sodium salt of Naringin, solve in 10000ml water for injection, adjust pH to 7~8 by hydrochloric acid, then filter with G3 sintered funnel, pour and seal. Sterilize at 100°C for 30min with flowing steam. So the injection of sodium salt of Naringin is given.

Example 42: Injection of sodium salt of Naringenin

Take 100g sodium salt of Naringenin, solve in 10L of water for injection, adjust pH to 7~8 with hydrochloric acid, then filter with G3 sintered funnel, pour and seal. Sterilize at 100°C for 30min with flowing steam. So the injection of sodium salt of Naringenin is given.

Example 43: Syrup of Naringin

Take 100g Naringin, add 650g cane sugar, mix equably, add 800ml distilled water, solve by heating water, filter and cold down, add some antiseptics, then dilute to 1000ml with distilled water. So the syrup of Naringin is given.

Example 44: Syrup of Naringenin

Take 100g Naringenin, add 500g cane sugar, mix equably, add 800ml distilled water, solve by heating water, filter and cold down, add some antiseptics, then dilute to 1000ml with distilled water. So the syrup of Naringenin is given.

Example 45: Powder for injection of sodium salt of Naringenin

Take 100g sodium salt of Naringenin, solve in 10000ml distilled water. Sterilize at 121°C for 15min, take out,

fill in 1ml ampoule flask under the circumstance of asepsis, freeze drying, then airproof under the circumstance of asepsis. So the powder for injection of sodium salt of Naringenin is given.

Example 46: Powder for injection of sodium salt of Naringin

Take 100g sodium salt of Naringin, solve in 10L of distilled water. Sterilize at 121°C for 15min, take out, fill in 1ml ampoule flask under the circumstance of asepsis, freeze drying, then airproof under the circumstance of asepsis. So the powder for injection of sodium salt of Naringin is given.

## Claims

1. A use of Naringenin, Naringin and salts thereof in preparation of pharmaceutical compositions for the treatment of cough and dispelling phlegm (mucus).

2. The use of claim 1, wherein an effective amount of Naringenin, Naringin and salts thereof is 0.1~500 mg/kg(avoirdupois) /day.

3. The use of claim 2, wherein the effective amount of Naringenin, Naringin and salts thereof is 1~100 mg/kg (avoirdupois )/day.

4. The use of any one of claims 1-3, wherein the type of said pharmaceutical compositions is selected from at least one of: tablet, syrup, capsule, powder for injection, injection and inhalant.

5. The use of any one of claims 1-4, wherein the pharmaceutical compositions are adapted to be given by oral access, injection or inhalation through lung.

6. The use of claim 1, wherein said Naringin and salts thereof have been synthesized according to the chemical method.

7. The use of claim 1, wherein said Naringin has been obtained by extraction from botanic materials containing Naringin.

8. The use of claim 1, wherein said Naringenin has been obtained by the hydrolyzation of Naringin.

9. The use of claim 1, wherein said Naringenin has been obtained by extraction from botanic materials containing Naringenin.

10. The use of claim 1, wherein said Naringenin has been obtained from the plant containing Naringenin by hydrolysation.

11. The use of any one of claims 1-5, wherein said the salt of Naringenin referred is a metal salt which is synthesized with Naringenin and relevant alkali substance, its molecular formula is: $C_{15}H_{(12-n)}O_5Y_n$, n=1-3, Y is a metal ion.

12. The use of claim 11, wherein said alkali substance is selected from at least one of: sodium hydroxide, potassium hydroxide, potassium hydroxide, sodium bicarbonate, kalium bicarbonate, sodium carbonate, and potassium carbonate; the Naringenin salt synthesized is sodium or potassium salt of Naringenin, its molecular formula is: $C_{15}H_{(12-n)}O_5Y_n$, n=1-3, Y is sodium or potassium ion.

13. The use of any one of claims 1-5, wherein said the salt of Naringin referred is metal salt is synthesized by Naringin and relevant alkali substance, which molecular formula is: $C_{27}H_{(32-n)}O_{14}Y_n$, n=1-2, Y is metal ion.

14. The use of claim 13, wherein said alkali substance is selected from at least one of: sodium hydroxide, potassium hydroxide, potassium hydroxide, sodium bicarbonate, kalium bicarbonate, sodium carbonate, and potassium carbonate; the Naringin salt synthesized is sodium or potassium salt of Naringin, its molecular formula is: $C_{27}H_{(32-n)}O_{14}Y_n$, n=1-2, Y is sodium or potassium ion.

## Patentansprüche

1. Verwendung von Naringenin, Naringin und Salzen davon zur Herstellung von pharmazeutischen Zusammensetzungen für die Behandlung von Husten und Entfernung von Schleim (Mucus).

2. Verwendung nach Anspruch 1, wobei eine wirksame Menge von Naringenin, Naringin und Salzen davon 0,1-500 mg/kg (Avoirdupois)/Tag beträgt.

3. Verwendung nach Anspruch 2, wobei die wirksame Menge an Naringenin, Naringin und Salzen davon 1-100 mg/kg (Avoirdupois)/Tag beträgt.

4. Verwendung nach irgendeinem der Ansprüche 1 bis 3, wobei der Typ der pharmazeutischen Zusammensetzungen ausgewählt ist aus mindestens einem von: Tablette, Sirup, Kapsel, Pulver für Injektionszwecke, Injektion und Inhalationsmittel.

5. Verwendung nach irgendeinem der Ansprüche 1 bis 4, wobei die pharmazeutischen Zusammensetzungen adaptiert sind, um durch orale Gabe, Injektion oder Inhalation durch die Lunge verabreicht zu werden.

6. Verwendung nach Anspruch 1, wobei das Naringin und die Salze davon nach einem chemischen Verfahren synthetisiert wurden.

7. Verwendung nach Anspruch 1, wobei das Naringin durch Extraktion aus botanischen Materialien, welche Naringin enthalten, erhalten wurde.

8. Verwendung nach Anspruch 1, wobei das Naringenin durch die Hydrolyse von Naringin erhalten wurde.

9. Verwendung nach Anspruch 1, wobei das Naringenin durch Extraktion aus botanischen Materialien, welche Naringenin enthalten, erhalten wurde.

10. Verwendung nach Anspruch 1, wobei das Naringenin aus der Naringenin enthaltenden Pflanze durch Hydrolyse erhalten wurde.

11. Verwendung nach irgendeinem der Ansprüche 1 bis 5, wobei das genannte Naringeninsalz ein Metallsalz ist, welches aus Naringenin und einer entsprechenden Alkalisubstanz synthetisiert wurde, dessen Molekülformel ist: $C_{15}H_{(12-n)}O_5Y_n$, n = 1-3, Y = ein Metallion.

12. Verwendung nach Anspruch 11, wobei die Alkalisubstanz ausgewählt ist aus mindestens einer von: Natriumhydroxid, Kaliumhydroxid, Kaliumhydroxid, Natriumhydrogencarbonat, Kaliumhydrogencarbonat, Natriumcarbonat und Kaliumcarbonat; das synthetisierte Naringeninsalz ein Natrium- oder Kaliumsalz von Naringenin ist, dessen Molekülformel ist:
$C_{15}H_{(12-n)}0O_5Y_n$, n = 1-3, Y = ein Natrium- oder Kaliumion.

13. Verwendung nach irgendeinem der Ansprüche 1 bis 5, wobei das genannte Salz von Naringin ein Metallsalz ist, das aus Naringin und einer entsprechenden Alkalisubstanz synthetisiert wurde, dessen Molekülformel ist: $C_{27}H_{(32-n)}O_{14}Y_n$, n = 1-2, Y = ein Metallion.

14. Verwendung nach Anspruch 13, wobei die Alkalisubstanz ausgewählt ist aus mindestens einer von: Natriumhydroxid, Kaliumhydroxid, Kaliumhydroxid, Natriumhydrogencarbonat, Kaliumhydrogencarbonat, Natriumcarbonat und Kaliumcarbonat; das synthetisierte Naringinsalz ein Natrium- oder Kaliumsalz von Naringin ist, dessen Molekülformel ist: $C_{27}H_{(32-n)}O_{14}Y_n$, n = 1-2, Y = ein Natrium- oder Kaliumion.

## Revendications

1. Utilisation de naringénine, naringine et de leurs sels pour la préparation de compositions pharmaceutiques pour le traitement de la toux et du flegme d’expectoration (mucus).

2. Utilisation selon la revendication 1, dans laquelle une quantité efficace de naringénine, de naringine et de leurs sels est de 0,1 à environ 500 mg/kg (corporel)/jour.

3. Utilisation selon la revendication 2, dans laquelle une quantité efficace de naringénine, de naringine et de leurs sels

est de 1 à environ 100 mg/kg (corporel)/jour.

4. Utilisation selon l'une quelconque des revendications 1 à 3, dans laquelle le type desdites compositions pharmaceutiques est choisi parmi au moins l'une des formes suivantes : comprimé, sirop, gélule, poudre pour injection, injection et inhalant.

5. Utilisation selon l'une quelconque des revendications 1 à 4, dans laquelle les compositions pharmaceutiques sont adaptées pour être administrées par voie orale, injection ou inhalation vers les poumons.

6. Utilisation selon la revendication 1, dans laquelle ladite naringine et ses sels ont été synthétisés selon le procédé chimique.

7. Utilisation selon la revendication 1, dans laquelle ladite naringine a été obtenue par extraction de substances botaniques contenant de la naringine.

8. Utilisation selon la revendication 1, dans laquelle ladite naringénine a été obtenue par hydrolyse de la naringine.

9. Utilisation selon la revendication 1, dans laquelle ladite naringénine a été obtenue par extraction de substances botaniques contenant de la naringénine.

10. Utilisation selon la revendication 1, dans laquelle ladite naringénine a été obtenue à partir de la plante contenant de la naringénine par hydrolyse.

11. Utilisation selon l'une quelconque des revendications 1 à 5, dans laquelle ledit sel de naringénine mentionné est un sel métallique qui est synthétisé avec de la naringénine et une substance alcaline appropriée, dont la formule moléculaire est : $C_{15}H_{(12-n)}O_5Y_n$, n = 1 à 3, Y est un ion métallique.

12. Utilisation selon la revendication 11, dans laquelle ladite substance alcaline est choisie parmi au moins l'un des composés suivants : hydroxyde de sodium, hydroxyde de potassium, hydroxyde de potassium, bicarbonate de sodium, bicarbonate de kalium, carbonate de sodium et carbonate de potassium ; le sel de naringénine synthétisé est un sel de sodium ou de potassium de la naringénine dont la formule moléculaire est : $C_{15}H_{(12-n)}O_5Y_n$, n = 1 à 3, Y est un ion sodium ou potassium.

13. Utilisation selon l'une quelconque des revendications 1 à 5, dans laquelle ledit sel de naringine mentionné est un sel métallique qui est synthétisé avec de la naringine et une substance alcaline appropriée, dont la formule moléculaire est : $C_{27}H_{(32-n)}O_{14}Y_n$, n = 1 à 2, Y est un ion métallique.

14. Utilisation selon la revendication 13, dans laquelle ladite substance alcaline est choisie parmi au moins l'un des composés suivants : hydroxyde de sodium, hydroxyde de potassium, hydroxyde de potassium, bicarbonate de sodium, bicarbonate de kalium, carbonate de sodium et carbonate de potassium ; le sel de naringine synthétisé est un sel de sodium ou de potassium de la naringine dont la formule moléculaire est : $C_{27}H_{(32-n)}O_{14}Y_n$, n = 1 à 2, Y est un ion sodium ou potassium.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6165984 A


### Non-patent literature cited in the description


- **Juhong Pills ; Chuanbei-Pipa Syrup ; Xiaoke-chuan Syrup.** *China Pharmacopoeia I,* 2000
- **Knekt et al.** *American Journal of Clinical Nutrition,* 2002, vol. 76 (3), 560-568
- **Tabac et al.** *American Journal of Respiratory and Critical Care Medicine,* 2001, vol. 164 (1), 61-64
- **Rosenmund.** *Ber.,* 1958, vol. 61, 2608
- **Zemlen ; Bognar.** *Ber.,* 1942, vol. 75, 648
- **Kazuo Ishii ; Takashi Furuta ; Yasuji Kasuya.** Determination of Naringin and Naringenin in human plasma by high-performance liquid chromatography. *Journal of Chromatography B,* 1996, vol. 683, 225-229